# EUROPÄISCHE PATENTSCHRIFT

(11) **EP 0 976 704 B1**
(45) Veröffentlichungstag und Bekanntmachung des Hinweises auf die Patenterteilung: **21.11.2001**
(21) Anmeldenummer: 99108731.3
(22) Anmeldetag: 03.05.1999
(51) Int. Cl.: C07C 17/156, C07C 19/045

(54) **Verfahren zur Energieschonung bei der Oxychlorierung von Ethylen**
Energy-saving process for oxychlorination of ethylene
Procédé permettant une économie d' énergie dans l'oxychloruration d' éthylène

(30) Priorität: 29.07.1998 DE 19834083
(43) Veröffentlichungstag der Anmeldung: 02.02.2000
(73) Patentinhaber: Krupp Uhde GmbH, 44141 Dortmund (DE)
(72) Erfinder: Benje, Michael Dr., 64289 Darmstadt (DE)
(74) Vertreter: Patentanwälte Meinke, Dabringhaus und Partner

(56) Entgegenhaltungen:
- DE-A- 4 029 314
- DE-A- 4 033 048
- DE-A- 4 132 030
- DE-A- 4 303 086
- FR-A- 2 314 907
- US-A- 4 873 384

## Beschreibung

Ausgehend von einem Stand der Technik, wie er beispielsweise in der DE-40 33 048-C2 und DE-41 32 030-A1 beschrieben ist, richtet sich die Erfindung auf ein Verfahren zur Energieschonung bei der Oxychlorierung.

Bekanntlich besteht das Reaktionsgemisch am Austritt eines Oxychlorierungsreaktors aus den Hauptkomponenten EDC (1,2-Dichlorethan), Wasser- und Kreislaufgas (Kohlendioxid, Stickstoff, Argon), wobei das Gemisch eine Temperatur um 220°C bei einem Druck von ca. 4,2 bar absolut aufweist. Da es zum Abrieb im Katalysator kommt, passiert der Reaktoraustrittstrom einen Katalysatorfeinstaubfilter und/oder Zyklone und wird in die Quenchkolonne eingeleitet, wobei die Kolonne dem Auswaschen und der Neutralisation nicht umgesetzten Chlorwasserstoffs im Reaktionsaustrittstrom dient und der alkalischen Zersetzung chlorierter Nebenprodukte, wie Chloral.

In der Quenchkolonne wird dem Reaktionsgemisch fühlbare Wärme entzogen, die Kopftemperatur der Kolonne beträgt ca. 102°C. Die Reaktionsprodukte Wasser und EDC werden in den der Kolonne nachgeschalteten Roh-EDC-Kondensatoren kondensiert und im Roh-EDC-Dekanter durch Schwerkraftabscheidung voneinander sowie vom Kreisgas getrennt. Das abgeschiedene Wasser wird zusammen mit Natronlauge (zur Neutralisation und Zersetzung von Nebenprodukten) als Rücklauf auf die Quenchkolonne mit einer Temperatur von ungefähr 40°C zurückgeführt. Am Sumpf der Quenchkolonne wird ein in etwa der Reaktionswassermenge entsprechender Strom abgezogen und einer Abwasserbehandlung zugeführt.

Um eine einwandfreie Funktion der Quenchkolonne zu gewährleisten, muß diese jederzeit mit einer ausreichenden und konstanten Flüssigkeitsmenge beaufschlagt werden, wobei sich der Kolonnenrücklauf gedanklich in zwei Teilkomponenten aufgliedern läßt, nämlich einmal in das Reaktionswasser, das mit dem Reaktoraustrittsstrom in die Kolonne eintritt und von den Roh-EDC-Kondensatoren kondensiert wird, und einmal in das Wasser aus der Kolonne, das mittels eines Teils der fühlbaren Wärme des Reaktoraustrittsstromes verdampft und ebenfalls in den Roh-EDC-Kondensatoren kondensiert wird. Diese letztere Komponente befindet sich daher durch kontinuierliche Verdampfung und nachfolgende Kondensation im Kreislauf, der größte Teil der fühlbaren Wärme des Reaktoraustritts wird durch Kühlwasser dissipiert.

Hier setzt die Erfindung an, deren Ziel es ist, möglichst viel Wärme zurückzugewinnen, um einen möglichst großen Teil der fühlbaren Wärme am Oxyreaktor für andere Zwecke nutzen zu können.

Mit einem Verfahren der eingangs bezeichneten Art wird diese Aufgabe gemäß der Erfindung dadurch gelöst, daß das aus dem Oxychlorierungsreaktor kommende Gasgemisch vor Eintritt in die Quenchkolonne gekühlt und die so gewonnene Wärme zur Vorwärmung des Ethylen-Kreisgas-Feedstromes eingesetzt wird.

Mit der Erfindung ist es möglich, die Erzeugung des Quenchrücklaufes und die Abkühlung des Reaktoraustrittstromes voneinander zu entkoppeln und damit einen großen Teil der fühlbaren Wärme am Austritt des Oxyreaktors zu anderen Zwecken heranzuziehen. Ein besonderer Vorteil der Erfindung liegt auch darin, daß das Quenchkopf-Kondensationssystem für eine geringere Leistung ausgelegt werden kann mit den sich daraus ergebenden Kostenvorteilen.

Um das vorhandene Wärmepotential des aus dem Oxychlorierungsreaktor kommenden Gasgemisches möglichst vollständig auszuschöpfen, wird in einer Ausgestaltung der vorliegenden Erfindung das Gasgemisch auf eine Temperatur dicht oberhalb seines vom Reaktionswassergehalt geprägten Taupunktes abgekühlt.

Damit wird sichergestellt, daß der überwiegende Teil des im Oxychlorierungsreaktor gebildeten Reaktionswassers als Dampf in die Quenchkolonne eingeleitet wird und im dampfförmigen Zustand zusammen mit dem EDC am Kopf der Quenchkolonne abgezogen werden kann, um nach seiner Kondensation und seiner Abtrennung im Dekanter als der erforderliche wässrige Quenchrücklauf genutzt werden zu können. Dies ist insbesondere vorteilhaft, weil im Falle der Taupunktunterschreitung ein Teil des Reaktionswassers als Flüssigkeit direkt in den Sumpf der Quenchkolonne einrieseln würde, wodurch ein erhöhter Natronlaugeverbrauch entstände, um den im Quenchsumpf notwendigen pH-Wert aufrechtzuerhalten.

Sofern das Gasgemisch mittels eines eingängigen, im Gleichstrom beaufschlagten Wärmeaustauschers gekühlt werden kann, so ist es vorteilhaft, sich mit der Austrittstemperatur des Gasgemisches dem Taupunkt so dicht anzunähern, daß eine Temperatur-Differenz von lediglich 5 K erhalten bleibt, was aus Gründen einer stabilen Prozeßführung eine durch die betriebliche Praxis gebotene Grenze darstellt.

Die vorliegende Erfindung ist allerdings nicht dahingehend beschränkt, daß sie ausschließlich bei Austrittstemperaturen oberhalb des Taupunktes eingesetzt werden kann, beispielsweise kann eine geänderte Reaktionsführung im Oxychlorierungsreaktor einen verminderten Natronlaugebedarf zur Folge haben, wodurch eine durch Taupunktunterschreitung flüssige Teilmenge des Reaktionswassers direkt in den Quenchwassersumpf hinein verrieselt werden könnte.

Weitere Ausgestaltungen der erfindungsgemäßen Verfahrensweise ergeben sich aus den Unteransprüchen.

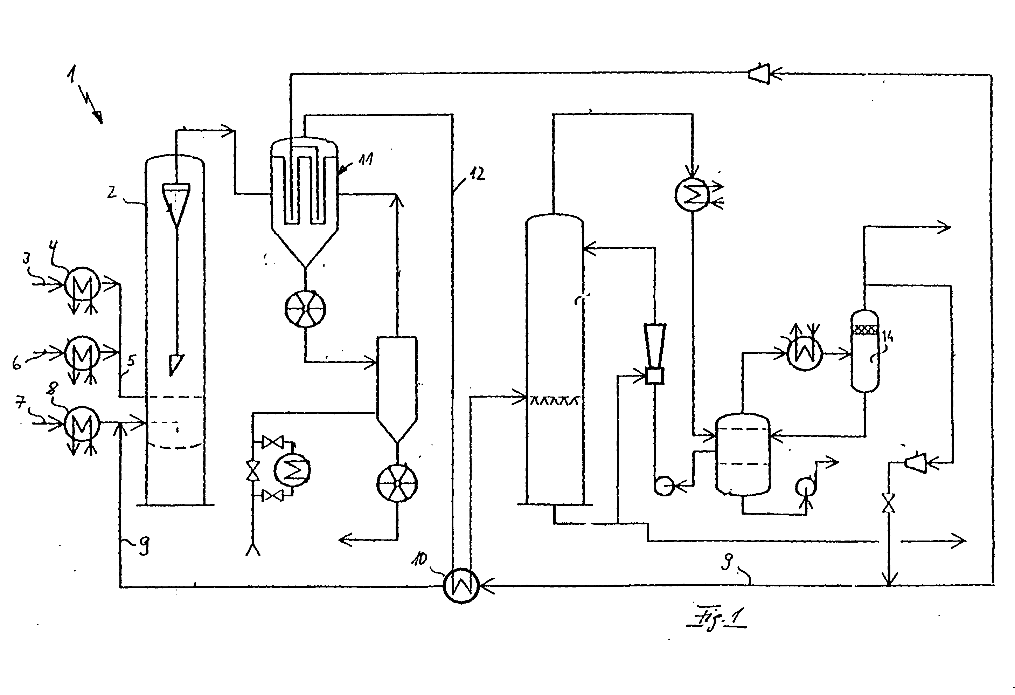

Weitere Vorteile, Einzelheiten und Merkmale der Erfindung ergeben sich aufgrund der nachfolgenden Beschreibung sowie anhand der Zeichnung. Diese zeigt in
- Fig. 1: ein Anlagenschaubild nach der Erfindung sowie in
- Fig. 2: ein leicht abgewandeltes Anlagenschaubild mit Hinweis auf Parameter eines weiter unten beschriebenen Beispieles.

Die allgemein mit 1 bezeichnete Anlage weist einen Reaktor 2 auf, dem über Leitungen 3 mit Vorwärmern 4 Chlorwasserstoff über die Leitung 5 zugeführt wird. Neben Sauerstoff (Leitung 6) wird Ethylen über die Leitung 7 und den Vorwärmer 8 ebenfalls dem Reaktor 2 zugeführt. Erkennbar wird über die Leitung 9 die Gasphase in den Reaktor 2 zurückgeführt, wobei die Gasphase über den Wärmetauscher 10 geleitet wird, der vom rohen 1,2-Dichlorethan-Gasstrom aus dem Kopf des Staubabscheiders 11 über die Leitung 12 beaufschlagt ist.

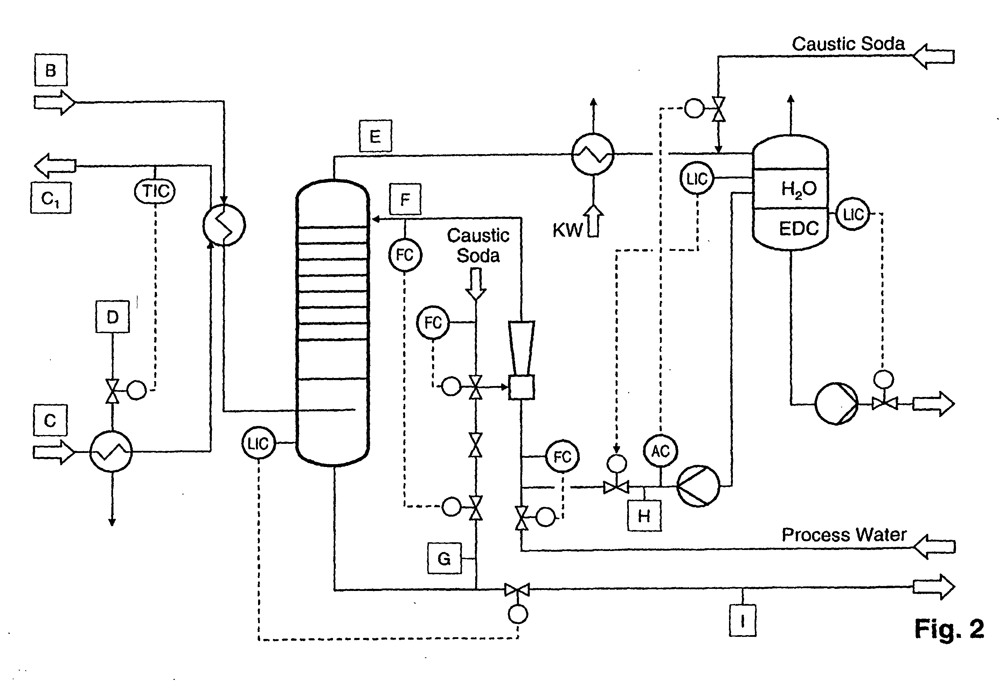

In Fig. 2 ist die Möglichkeit dargestellt, z.B. die Zumischung des Quenchsumpfabzuges über eine von der Wasserphase aus dem Roh-EDC-Dekanter getriebene Wasserstrahlpumpe 13 vornehmen zu lassen. In Bezugnahme auf die nachfolgend wiedergegebenen Beispielparamter sind in Fig. 2 in kleinen Quadraten Großbuchstaben aufgeführt.

### Wärmebilanz bei Oxyquenchen bei Feed-Vorwärmung

- A:: 43561 Kg/h
3128 Kg/h
H₂OT = 160°C
Δ H (160 → 101°C) ∼ 750 kW
- B:: Austritt Oxyreaktor
43540 Kg/h
T = 220°C
p ≈ 4,2 bar a
- C:: Ethylen-Kreislaufgas
T ≈ 57°C
p ≈ 6.2 bar a
C1 Ethylen-Kreisgas T = 150°C
p ≈ 6 bar a
- D:: Dampf
10 bar a 180°C
- E:: 44329 Kg/h
3896 Kg/hH₂O
T ≈ 101°C
- F:: 5155 Kg/h
5089 Kg/hH₂O
T ≈ 56°C
- G:: 1259 Kg/h
T ≈ 105°C
- H:: 3896 Kg/hH₂O
T ≈ 40°C
- I:: 3347 Kg/h
T ≈ 105°C.

Wie sich aus Fig. 2 und den obigen Zahlenangaben ergibt, wird der Reaktoraustritt in einem eingängigen Wärmetauscher auf 160°C abgekühlt und befindet sich damit noch oberhalb des Taupunktes. Der Feed-Strom wird bei dieser Variante auf 150°C aufgewärmt.

Die Temperatur des Quenchkopfstromes beträgt weiterhin 101°C, allerdings wird nun in der Kolonne weniger Wasser verdampft. Dies führt zu einer etwa 12 % geringeren Leistung des Quenchkopfkondensators. Um einen ausreichenden Quenchrückfluß aufrechtzuerhalten, wird ein Teil des Quenchsumpfabzuges zugeführt und mit der Wasserphase aus dem Roh-EDC-Dekanter gemischt. Durch den Einsatz einer Wasserstrahlpumpe 13 kann als Treibstrahl die Wasserphase aus dem Roh-EDC-Dekanter eingesetzt werden. Bei Schwankungen wird dieser von einer Mengenregelung durch Zugabe vom Prozeßwasser konstant gehalten. Die gesamte Rücklaufmenge wird durch Mittelzirkulierung des angesaugten Stromes durch eine weitere Mengenregelung eingestellt. Zum angesaugten Strom wird eine fest eingestellte Menge Natronlauge dosiert, wie sich dies aus Fig. 2 ergibt. Der Quenchsumpf kann damit in jeden pH-Bereich gefahren werden.

Bei dem in Fig. 2 und dem in der obigen Tabelle angegebenen Beispiel ergibt sich eine erhebliche Betriebskosteneinsparung.

## Patentansprüche

1. Verfahren zur Energieschonung bei der Oxychlorierung von Ethylen,
**dadurch gekennzeichnet,**
**daß** das aus dem Oxychlorierungsreaktor kommende Gasgemisch vor Eintritt in die Quenchkolonne gekühlt und die so gewonnene Wärme zur Vorwärmung des Ethylen-Kreisgas-Feedstromes eingesetzt wird.

2. Verfahren nach Anspruch 1,
**dadurch gekennzeichnet,**
**daß** das aus dem Oxychlorierungsreaktor kommende Gasgemisch vor Eintritt in die Quenchkolonne auf eine Temperatur 5K oberhalb des Taupunktes gekühlt wird.

3. Verfahren nach Anspruch 1 oder 2,
**dadurch gekennzeichnet,**
**daß** ein Teil des Quenchsumpfabzuges dem Quenchrückfluß zugeführt wird.

4. Verfahren nach Anspruch 3,
**dadurch gekennzeichnet,**
**daß** der zugeführte Teil des Quenchsumpfabzuges mit der Wasserphase aus dem Roh-EDC-Dekanter gemischt wird.

5. Verfahren nach einem der vorangehenden Ansprüche,
**dadurch gekennzeichnet,**
**daß** die Zumischung des Quenchsumpfabzuges über eine von der Wasserphase aus dem Roh-EDC-Dekanter getriebene Wasserstrahlpumpe vorgenommen wird.

6. Verfahren nach einem der vorangehenden Ansprüche,
**dadurch gekennzeichnet,**
**daß** dem zugeführten Quenchsumpfabzug Natronlauge zudosiert wird.

## Claims

1. A method of energy conservation in the oxychlorination of ethylene, **characterised in that** the gas mixture coming from the oxychlorination reactor is cooled before passing into the quench column and the heat obtained **in that** way is used for preheating the ethylene-cycle gas feed flow.

2. A method according to claim 1 **characterised in that** the gas mixture coming from the oxychlorination reactor is cooled to a temperature 5K above the dew point before passing into the quench column.

3. A method according to claim 1 or claim 2 **characterised in that** a part of the quench sump discharge is fed to the quench return flow.

4. A method according to claim 3 **characterised in that** the part of the quench sump discharge fed to the quench return flow is mixed with the water phase from the crude EDC decanter.

5. A method according to one of the preceding claims **characterised in that** admixing of the quench sump discharge is effected by way of a water jet pump operated by the water phase from the crude EDC decanter.

6. A method according to one of the preceding claims **characterised in that** caustic soda is added to the fed quench sump discharge.

## Revendications

1. Procédé pour économiser l'énergie lors de l'oxychloration de l'éthylène,
**caractérisé en ce que** le mélange de gaz provenant du réacteur d'oxychloration est refroidi avant son entrée dans la colonne d'arrêt de réaction et la chaleur ainsi gagnée est utilisée pour le préchauffage du courant d'alimentation éthyléne-gaz de cycle.

2. Procédé selon la revendication 1,
**caractérisé en ce que** le mélange de gaz provenant du réacteur d'oxychloration est refroidi avant son entrée dans la colonne d'arrêt de réaction à une température située 5K au-dessus du point de rosée.

3. Procédé selon la revendication 1 ou 2,
**caractérisé en ce qu'**une partie du soutirage du fond de colonne est ajoutée au courant de retour à la colonne.

4. Procédé selon la revendication 3,
**caractérisé en ce que** la partie renvoyée du soutirage du fond de colonne est mélangée avec la phase aqueuse provenant du décanteur de EDC brut.

5. Procédé selon l'une quelconque des revendications précédentes,
**caractérisé en ce que** le mélange du soutirage du fond de colonne est effectué au moyen d'une pompe à jet d'eau entraînée par la phase aqueuse provenant du décanteur EDC brut.

6. Procédé selon l'une quelconque des revendications précédentes,
**caractérisé en ce qu'**une quantité dosée de lessive de soude est ajoutée au soutirage ajouté du fond de colonne.
